# EUROPÄISCHE PATENTSCHRIFT

(11) **EP 0 884 020 B1**
(45) Veröffentlichungstag und Bekanntmachung des Hinweises auf die Patenterteilung: **27.08.2003**
(21) Anmeldenummer: 98110325.2
(22) Anmeldetag: 05.06.1998
(51) Int. Cl.: A61B 1/04

(54) **Endoskop mit einer Bildausrichtung für endoskopische Video-Displays**
Endoscope with image alignment for endoscopic video displays
Endoscope avec alignement de l'image pour afficheurs vidéo endoscopiques

(30) Priorität: 06.06.1997 US 870792
(43) Veröffentlichungstag der Anmeldung: 16.12.1998
(73) Patentinhaber: Karl Storz Imaging Inc., Goleta, CA 93117 (US)
(72) Erfinder: Mattsson-Boze, Daniel, Goleta, CA 93117 (US); Chatenever, David, Santa Barbara, CA 93105 (US)
(74) Vertreter: Weller, Wolfgang, Dr.rer.nat.

(56) Entgegenhaltungen:
- EP-A- 0 501 088
- DE-A- 19 609 034

## Beschreibung

Die Erfindung betrifft ein Endoskop mit einem Bildleitsystem, einem Gestell und einer Kamera, wobei das Bildleitsystem eine mittige optische Achse aufweist und relativ zum Gestell ein Bild mit einer Hochachse und einer Querachse erzeugt, wobei die Kamera einen sensitiven Bereich aufweist, der auf die optische Achse zentriert ist und wobei die Kamera relativ zu sich selbst eine Hochachse und eine Querachse aufweist.

Endoskope weisen eine langerstreckte rohrförmige Struktur auf und dienen dazu, in eine Körperhöhle eingebracht zu werden, um darin Beobachtungen durchzuführen. Ein Endoskop weist eine Beobachtungsoptik mit einer Objektivlinse am distalen Ende auf. Die Beobachtungsoptik weist ein Bildleitsystem auf. Bei starren Endoskopen besteht dieses aus einer Reihe voneinander beabstandeten Linsen. Bei flexiblen Endoskopen besteht es aus einem Bündel an dünnen optischen Fasern, die kohärent zueinander zusammengesetzt sind, um das Bild zu leiten. Die Erfindung ist bei beiden Arten an Bildleitsystemen einsetzbar.

Am proximalen Ende des Bildleitsystems ist ein Okular angeordnet, das ein für das menschliche Auge unmittelbar ersichtliches virtuelles Bild erzeugt. Oftmals ist eine Kamera an dem Endoskop angebracht. Eine derartige Kamera kann bspw. ein CCD-(Charge-Coupled-Device)-Chip sein, dieser lichtempfindliche Chip setzt optische Signale in elektrische Signale um. Das Bild wird somit in ein Signal für ein Video-Display umgesetzt.

Obwohl manche Operateure direkt über das Okular durch das Endoskop schauen, und dies oftmals durchführen, sind diese immer mehr damit vertraut, eine an das Endoskop angebrachte Videokamera einzusetzen und das Bild über einen Videoschirm zu beobachten. Bei operativen oder diagnostischen Vorgängen manipuliert der Operateur das Endoskop. Er kann es kippen, er kann es hineinbewegen oder herausziehen, und er kann es auch um dessen mechanische Achse drehen. Werden diese Manipulationen mit einem Endoskop mit einer daran angebrachten CCD-Kamera durchgeführt, liefert die Kamera originalgetreu das, was für sie erkennbar ist, wobei deren Hochachse als die Hochachse des Bildes auf dem Display dargestellt wird. Das bedeutet, ist die Kamera starr mit dem Endoskop verbunden und wird die Endoskopkamera um dessen mechanische Achse gedreht, so bewegt sich das auf dem Monitor dargestellte Bild proportional und in die entgegengesetzte Richtung wie die Endoskopkamera. Ein Drehen der Endoskopkamera um einen Winkel von 45° in Uhrzeigerrichtung verursacht ein Drehen des Bildes auf dem Monitor um 45° entgegen der Uhrzeigerrichtung.

Dies ist das eigentliche Problem. Wird das Bild auf einem Bildschirm dargestellt und wird das Endoskop um dessen Achse gedreht, müßte der Operateur seinen Kopf neigen, um diesem zu folgen. Der Operateur steht jedoch aufrecht und das sich drehende Bild ist für ihn verwirrend. Was er jedoch tatsächlich auf dem Bildschirm sehen möchte, ist ein Bild, das so ausgerichtet ist wie das Bild, das er sehen würde, wenn er sich innerhalb des Lichtpfades befände, nämlich hochstehend mit derselben hoch ausgerichteten Orientierung. Anders ausgedrückt, er bevorzugt dasjenige zu sehen, was er sehen würde, wenn er direkt in das Endoskop hineinschauen würde, anstatt den Bildschirm zu beobachten. Dies ist jedoch unmöglich, falls die Kamera fest am Endoskop angebracht ist und mit diesem gedreht wird, während der Operateur sich dabei nicht mitdreht.

Bei einer bekannten Anordnung von Endoskop und Kamera ist die Kamera üblicherweise lösbar und drehbar mit dem Endoskop verbunden. Bei dieser Anordnung kann das gedrehte Bild auf dem Bildschirm dadurch ausgerichtet wird, daß nur die Kamera in entgegengesetztem Sinne derart ausgerichtet wird, daß deren Ausrichtung aufrechtstehend ist. Alternativ dazu kann man derartige Bilddrehzustände dadurch verhindern, daß man die Kamera in ihrer aufrechten Stellung hält und nur das Endoskop dreht.

Es wurde bereits Vorschläge gemacht, um die Kamera von der Beobachtungsoptik derart zu entkoppeln, daß die Kamera unabhängig davon gedreht werden kann, wobei ein Pendel eingesetzt wird, um diese vertikal auszurichten , siehe z.B. EP-A-0 501 088. Diese scheinbar empfindliche Art und Weise steht jedoch in Konflikt mit den Bedingungen, die bei der Verwendung des Instrumentes bestehen.

Endoskope werden in engen Bewegungsbereichen eingesetzt und deren proximale Enden müssen so klein wie möglich und so unbeeinträchtigt wie möglich sein. Physikalische Wechselwirkungen mit der Umgebung und mit der Hand des Operateurs müssen ausgeschlossen oder größtmöglichst minimalisiert werden. Ein dafür einsetzbares Pendel muß eine beachtliche Masse und einen beachtlichen Winkelbereich belegen, um durchgehend zu arbeiten, wodurch das Instrument erweitert wird. Darüber hinaus verläuft die Rotationsachse des Pendels nicht mehr länger horizontal, wenn das Endoskop gekippt wird. Dazu müssen Lager vorgesehen werden, um das Pendel zu stützen und der Anteil der Gravitationskraft, die auf das Pendel einwirkt, ist reduziert. Noch ungünstiger ist, falls die Neigung des Endoskopes sehr steil ist, so erhält das mechanische Pendel keine ausreichende Kraft mehr, um die Vertikalausrichtung zu suchen.

Es bestehen jedoch manchmal Gründe dafür, das Endoskop derart anzubringen, daß es bezüglich der Kamera nicht gedreht werden kann. Oder, alternativ dazu, kann es wünschenswert sein, die CCD-Kamera in das Endoskopgehäuse einzubetten. Unter diesen Umständen ist es nicht möglich, die Kamera manuell bezüglich des Endoskopes zu drehen, so daß andere Mittel notwendig sind, um das gezeigte Bild auszurichten. Darüber hinaus ist es wünschenswert, diese Drehung des Bildes automatisch durchzuführen, so daß, unabhängig von der physikalischen Ausrichtung der Endoskopkamera im Raum, das gezeigte Bild eines Gegenstandes jeweils korrekt bezüglich des Bezugssystems des Beobachters ausgerichtet ist.

Ein Drehen des Bildes durch elektronische Manipulation erscheint als eine geeignete Lösung. Dies steht jedoch im Konflikt mit dem Bildformat der Kamera und deren Display. Ein derartiges Drehen kann vom dargestellten Bild Informationen, die im Bereich der Ecken und Kanten des beobachteten Bereiches liegen, wegnehmen, die jedoch von Interesse für den Operateur sind. Dieses Problem kann dadurch sichtbar gemacht werden, daß eine Fotografie in einem Bilderrahmen gedreht wird. Darüber hinaus erfordert dies exotische und teure Sensorvorrichtungen.

Es ist daher Aufgabe der vorliegenden Erfindung, die Drehstellung der das Bild erzeugenden Vorrichtung so auszurichten, daß dessen Hochachse in einer Vertikalebene bezüglich der Betrachtungsperson liegt.

Erfindungsgemäß wird die Aufgabe dadurch gelöst, daß ein Beschleunigungsmesser vorgesehen ist, daß ferner eine Anbringung vorgesehen ist, über die der Beschleunigungsmesser und die Kamera derart am Gestell anbringbar sind, daß diese simultan um die optische Achse drehbar sind, daß ferner ein in zwei Richtungen wirkender Antrieb vorgesehen ist, um die Kamera und den Beschleunigungssensor um die mittige optische Achse zu drehen, und daß eine Servosteuerung vorgesehen ist, die auf ein Signal vom Beschleunigungsmesser anspricht, so daß der Antrieb die Kamera und den Beschleunigungsmesser derart dreht, daß die Hochachse der Kamera in einer Vertikalebene liegt, die die mittige optische Achse enthält, so daß, wenn ein von der Kamera stammendes Bild auf einem Video-Display erscheint, die Querachse des Bildes mit der horizontal angeordneten Querachse des Video-Displays zusammenfällt.

Das endoskopische Kamerasystem beinhaltet einen Kamerakopf, der ein inneres Bildabtastsystem (wie bspw. einen CCD-Chip) aufweist, ferner eine Kameraprozeßeinheit, die die Signale vom Kamerakopf in Standardvideosignale (wie bspw. NTSC) verarbeitet, die geeignet sind, einem standardmäßigen Fernsehmonitor zugeführt zu werden, und außerdem ein Video-Display um das über die Kamera von einem Endoskop erhaltene Bild darzustellen. Der CCD-Chip ist am Gestell des Kamerakopfes drehbar befestigt, und zwar derart, daß dessen mittige Achse mit der des optischen Eintritts in den Kamerakopf zusammenfällt. Erfindungsgemäß ist ein Beschleunigungsmesser mit dem CCD-Chip verbunden, der auf die Erdanziehungskraft anspricht. Der Beschleunigungsmesser erzeugt ein Signal, das proportional zu seiner Position relativ zur Vertikalen im Standardgravitationsfeld ist. Das Signal ist an seinem Maximum, wenn dessen Hochachse vertikal ist. Die Servosteuerung dient dazu, dieses Signal zu maximieren, indem diese einen Servomotor derart aktiviert, daß er den CCD-Chip so dreht, um den Maximalwert zu erhalten. Mit dieser Anordnung ist die vom Video-Display dargestellte Ansicht jeweils "in Ausrichtung", und zwar in einer aufrechten Ausrichtung unabhängig davon, ob es von einem stehenden oder sitzenden Operateur betrachtet wird.

Es versteht sich, daß die vorstehend genannten und die nachstehend noch zu erläuternden Merkmale nicht nur in den angegebenen Kombinationen, sondern auch in anderen Kombinationen oder ein Alleinstellung einsetzbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine Seitenansicht eines Endoskops, das für die Erfindung Einsatz findet,
- Fig. 2: eine Draufsicht auf das rechte Ende von Fig. 1,
- Fig. 3: eine Seitenansicht, teilweise schematisiert, eines Gestells für die Bildaufnahme- und die Ausrichtungsabschnitte der Erfindung, das auch zum Anbringen des Endoskops dient,
- Fig. 4: eine Seitenansicht der Bildaufnahme und Ausrichtabschnitte, und
- Fig. 5: ein schematisches Diagramm, das die Steuerung der Erfindung erläutert.

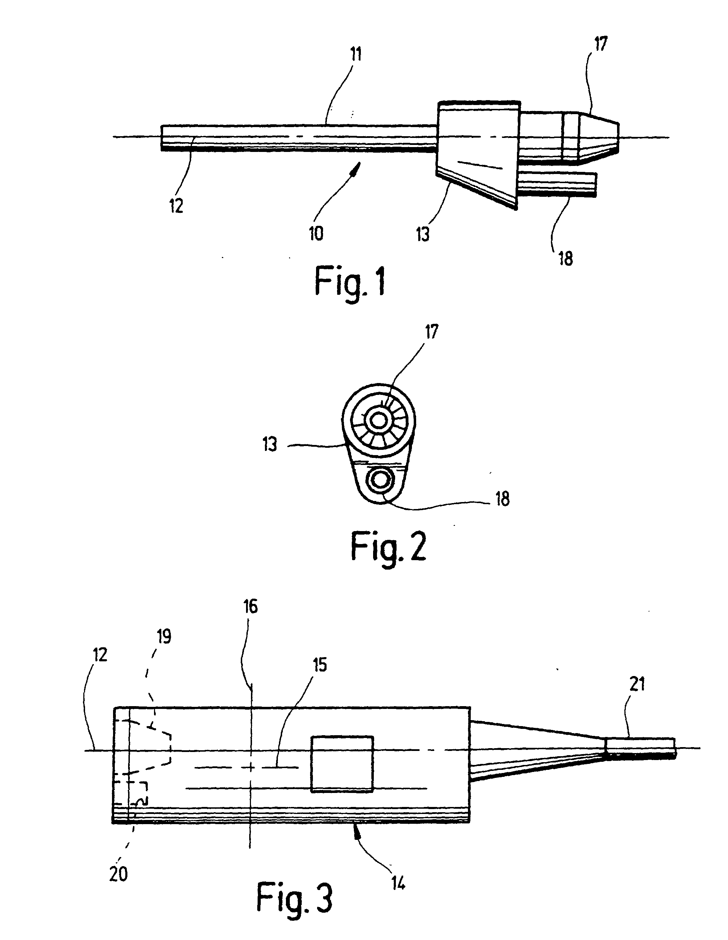

In Fig. 1 ist schematisch ein Endoskop 10 dargestellt. Das Endoskop 10 weist einen Schaft 11 auf, der diejenigen Bauelemente aufnimmt, die üblicherweise vorgesehen sind. Ein Bildleitsystem, wie bspw. eine Reihe an Linsen oder ein kohärentes optisches Faserbündel, ein Lichtleiter und ein Instrumentenkanal sind Beispiele dafür. Sie sind nicht näher dargestellt, da diese für das Verständnis der Erfindung nicht notwendig sind. Das Bildleitsystem hat eine mittige optische Achse 12. Das Endoskop 10 kann andauernd oder lösbar mit einem Anschlußstück 13 verbunden sein.

Das Anschlußstück 13 ist lösbar an einem Gestell 14 (siehe Fig. 3) anbringbar.

Das Gestell 14 selbst weist eine Querachse 15 auf, die horizontal verläuft, falls sich das Gestell 14 in seiner aufrechten Stellung befindet, und weist ferner eine Hochachse 16 auf, die bezüglich des Gravitationsfeldes vertikal verläuft, wenn sich das Gestell in seiner aufrechten Stellung befindet. Die Achsen 15 und 16 verlaufen senkrecht zueinander.

Wie aus den Fig. 1 bis 3 zu entnehmen, weist das Anschlußstück 13 zwei Vorsprünge 17, 18 auf, die jeweils in entsprechende Aufnahmen 19, 20 des Gestells 14 einsteckbar sind. Der Vorsprung 17 stellt die Kulmination des Bildpfades durch das Endoskop dar. Dieser kann mit diesem in Ausrichtung stehen oder er kann durch Prismen, Spiegel oder andere Mittel versetzt sein, um die optische Achse 12 seitlich zu verschieben. Ist das Endoskop 10 zutreffend am Gestell 14 befestigt, so wird das Bild in das Gestell 14 eingeleitet, und zwar so wie es ersichtlich ist. Dies stellt ein Beispiel eines Aufbaus dar, bei dem das Endoskop nicht relativ zu dem Gestell verdreht werden kann, in dem die Kamera eingebaut ist. Soll das Bild in einer "aufrechten" Stellung gehalten werden, muß die Kamera selbst gedreht werden.

Der Vorsprung 18 erhält Beleuchtungslicht von einer hier nicht dargestellten Quelle, das zu diesem über ein Kabel mit optischen Fasern geleitet werden kann, das Bestandteil eines Kabels 21 sein kann, das mit dem Gestell 14 verbunden ist. Falls gewünscht, kann ein separates Lichtkabel vorgesehen sein und mit dem Gestell 14 geeignet verbunden werden. Diese Anordnung der Vorsprünge 17, 18 stellt eine korrekte Ausrichtung des Endoskops 10 relativ zum Gestell 14 sicher.

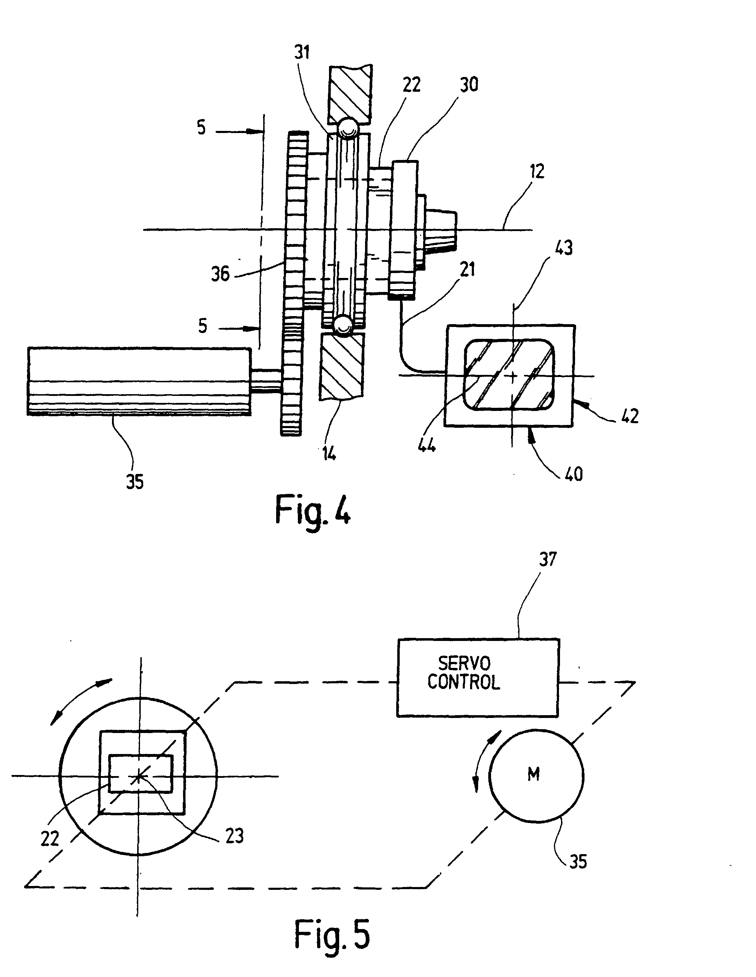

Am Gestell 14 ist drehbar ein CCD-Chip 22 (manchmal auch als "Kamera" bezeichnet) angebracht. Dessen Mittelpunkt 23 (siehe insbesondere Fig. 5) liegt auf der optischen Achse 12 des optischen Systems. Die Kamera ist um die optische Achse 12 drehbar, und zwar relativ zum optischen System und zum Gestell 14. Die Kamera weist eine eigene Querachse 24 und eine Hochachse 25 auf.

Am Gestell 14 ist außerdem ein Beschleunigungsmesser 30 drehbar befestigt, und zwar derart, daß er sich mit der Kamera dreht. Am besten ist der Beschleunigungsmesser 30 direkt mit dem CCD-Chip 22 verbunden. Beide sind drehbar am Gestell 14 angebracht. Sind beide miteinander verbunden, so kann eine einzige Lagerung 31 dazu dienen, beide drehbar zu lagern.

Der Beschleunigungsmesser 30 ist in der Lage, sensitiv auf Veränderungen der Gravitationskraftkomponenten anzusprechen, denen er ausgesetzt ist. Integrierte Beschleunigungsmesser des Typs, wie sie in Airbags eingesetzt werden, sind für diesen Zweck geeignet. Eine Analogvorrichtung ADXL-05, die einen mikrobearbeiteten einseitig angebrachten Ausleger aus Silizium aufweist, der zwischen zwei Elektroden aufgehängt ist, ist ein Beispiel für einen geeigneten Beschleunigungsmesser. An den beiden Elektroden kann ein Wechselspannungssignal angelegt werden und die detektierte Nähe des Auslegers zu den beiden Elektroden erzeugt ein Servosignal entsprechend einer Orientierung aus der Vertikalen heraus.

Es sei an dieser Stelle angemerkt, daß das Endoskop im Gebrauch in alle Richtungen geneigt werden kann, so daß der Beschleunigungsmesser oftmals auf eine Komponente der vertikalen Gravitationskraft anspricht, die beträchtlich geringer ist als deren Maximalwert. Ist bspw. die optische Achse um 60° geneigt, so ist die Vertikalkomponente der Gravitation, auf die der Beschleunigungsmesser anspricht, um das Bild aufrecht zu halten, wesentlich geringer als die maximale Gravitationskraft. Es ist ein Vorteil dieser Vorrichtung, die kein Pendel aufweist, daß sie über einen großen Winkelbereich zutreffend anspricht, in dem die Vertikalkomponente der Gravitation ziemlich klein ist.

Ein in zwei Richtungen arbeitender Servomotor 35 ist über einen Getriebezug 36 antreibend mit der Kamera und mit dem Beschleunigungsmesser 30 verbunden. Dieser spricht auf ein Signal der Servosteuerung 37 dahingehend an, die Kamera und den Beschleunigungsmesser 30 so zu drehen, daß der Beschleunigungsmesser 30 ein maximales Signal erzeugt. Das bedeutet, das stärkste Signal relativ zu den Signalen, die erzeugt werden, wenn der Beschleunigungsmesser in irgendeiner Richtung gedreht wird. Die Servosteuerung 37 erzeugt ihr Signal entsprechend dem Output des Beschleunigungsmessers 30.

Die Kamera überträgt ihr Signal über Leiter im Kabel 21 zu einem Video-Display 40. Das Display ist üblicherweise in einer Halterung oder in einer Konsole 42 an einer Wand oder einer Decke angebracht. Sein Bildschirm weist eine Hochachse 43 und eine Querachse 44 auf. Diese Achsen werden im allgemeinen als vertikal und horizontal angesehen. Befindet sich die Kamera in ihrer aufrechten Position, so fallen die Achsen des Displays mit den Achsen der Kamera zusammen. Es ist zu erkennen, daß ein Drehen des CCD-Chips 22 derart ist, daß dessen Achsen in nominal horizontaler und vertikaler Ausrichtung verbleiben, dies zu einer gleichen Ausrichtung des Bildes auf dem Bildschirm führt, wie auch immer die Stellung des Endoskops sein mag. Demzufolge bleibt der Operateur relativ zur Operationsstelle gleich räumlich orientiert. Er hat keine Anstrengungen dahingehend auszuführen, sich selbst auf ein Bild, das sich auf dem Display dreht, auszurichten.

Weiter vorteilhaft ist, daß diese Anordnung den gesamten Bereich des Blickfeldes zeigt, das der Kamera zur Verfügung steht. Das Abbildungsverhältnis von Bildschirm und Kamera ist gleich. Werden Bilder gedreht, so können Ecken und einige Kanten des Bildschirmes leer sein. So können möglicherweise wichtige Informationen aus den Eckbereichen der Kamera verlorengehen. Bei der vorliegenden Erfindung ist dieses Risiko nicht vorhanden.

## Patentansprüche

1. Endoskop, mit einem Bildleitsystem, einem Gestell (14) und einer Kamera, wobei das Bildleitsystem eine mittige optische Achse (12) aufweist und relativ zum Gestell (14) ein Bild mit einer Hochachse und einer Querachse erzeugt, wobei die Kamera einen sensitiven Bereich aufweist, der auf die optische Achse (12) zentriert ist, und wobei die Kamera relativ zu sich selbst eine Hochachse und eine Querachse aufweist, **dadurch gekennzeichnet, daß** ein Beschleunigungsmesser (30) vorgesehen ist, daß ferner eine Anbringung vorgesehen ist, über die der Beschleunigungsmesser (30) und die Kamera derart am Gestell (14) anbringbar sind, daß diese simultan um die optische Achse (12) drehbar sind, daß ein in zwei Richtungen wirkender Antrieb (35) vorgesehen ist, um die Kamera und den Beschleunigungsmesser (30) um die mittige optische Achse (12) zu drehen, und daß ein Servosteuerung (37) vorgesehen ist, die auf ein Signal vom Beschleunigungsmesser (30) anspricht, so daß der Antrieb die Kamera und den Beschleunigungsmesser (30) derart dreht, daß die Hochachse der Kamera in einer Vertikalebene liegt, die die mittige optische Achse (12) enthält, so daß, wenn ein von der Kamera stammendes Bild auf einem Video-Display (40) erscheint, die Querachse des Bildes mit der horizontal angeordneten Querachse (44) des Video-Displays (40) zusammenfällt.

2. Endoskop nach Anspruch 1, **dadurch gekennzeichnet, daß** die Kamera ein CCD-Chip (22) ist.

3. Endoskop nach Anspruch 2, **dadurch gekennzeichnet, daß** der Beschleunigungsmesser (30) mit dem CCD-Chip (22) verbunden ist.

4. Endoskop nach Anspruch 1, **dadurch gekennzeichnet, daß** die Kamera eine Videokamera ist.

5. Endoskop nach einem der Ansprüche 1 bis 4, **dadurch gekennzeichnet, daß** der Beschleunigungsmesser (30) einen zwischen einem Elektrodenpaar angeordneten Ausleger aus Silizium aufweist, dessen Stellung relativ zu den Elektroden das Signal für die Servosteuerung (37) erzeugt.

## Claims

1. Endoscope having an image guidance system, a frame (14) and a camera, the image guidance system having a central optical axis (12) and generating relative to the frame (14) an image with an upright axis and a transverse axis, the camera having a sensitive area which is centered on the optical axis (12), and the camera having relative to itself an upright axis and a transverse axis, **characterized in that** an accelerometer (30) is provided, **in that** further a mounting is provided via which the accelerometer (30) and the camera can be mounted on the frame (14) in such a way that these can be rotated simultaneously about the optical axis (12), **in that** a drive (35) acting in two directions is provided in order to rotate the camera and the accelerometer (30) about the central optical axis (12), and **in that** a servo control (37) is provided which responds to a signal from the accelerometer (30) such hat the drive rotates the camera and the accelerometer (30) in such a way that the upright axis of the camera lies in a vertical plane which contains the central optical axis (12) such that when an image coming from the camera appears on a video display (40) the transverse axis of the image coincides with the horizontally arranged transverse axis (44) of the video display (40).

2. Endoscope of Claim 1, **characterized in that** the camera is a CCD chip (22).

3. Endoscope of Claim 2, **characterized in that** the accelerometer (30) is connected to the CCD chip (22).

4. Endoscope of Claim 1, **characterized in that** the camera is a video camera.

5. Endoscope of anyone of Claims 1 to 4, **characterized in that** the accelerometer (30) has a horizontal arm arranged between an electrode pair and made from silicon whose position relative to the electrodes generates the signal for the servo control (37).

## Revendications

1. Endoscope avec un système de conduite d'image, un bâti (14) et une caméra, dans lequel le système de conduite d'image comprend un axe optique central (12) et engendre relativement au bâti (14) une image avec un axe vertical et un axe transversal, dans lequel la caméra comprend une zone sensitive, qui est centrée sur l'axe optique (12), et dans lequel la caméra présente par rapport à elle-même un axe vertical et un axe transversal, **caractérisé en ce qu'**un accéléromètre (30) est prévu, **en ce que** en outre un montage est prévu, par lequel f'accéléromètre (30) et la caméra peuvent être montés sur le bâti (14) de telle sorte que ces derniers puissent tourner simultanément autour de l'axe optique (12), **en ce qu'**un entraînement (35) agissant dans deux directions est prévu pour tourner la caméra et l'accéléromètre (30) autour de l'axe optique central (12), et **en ce qu'**une servocommande (37) est prévue, qui réagit à un signal de l'accéléromètre (30), de telle sorte que l'entraînement tourne la caméra et l'accéléromètre (30) de manière à ce que l'axe vertical de la caméra se trouve dans un plan vertical qui contient l'axe optique central (12), de telle sorte que, quand une image en provenance de la caméra apparaît sur un affichage vidéo (40), l'axe transversal de l'image vient en coïncidence avec l'axe transversal disposé horizontalement (44) de l'affichage vidéo (40).

2. Endoscope selon la revendication 1, **caractérisé en ce que** la caméra est une puce CCD (22).

3. Endoscope selon la revendication 2, **caractérisé en ce que** l'accéléromètre (30) est relié à la puce CCD (22).

4. Endoscope selon la revendication 1, **caractérisé en ce que** la caméra est une caméra vidéo.

5. Endoscope selon l'une quelconque des revendications 1 à 4, **caractérisé en ce que** l'accéléromètre (30) comporte une flèche en silicium disposée entre une paire d'électrodes, dont la position par rapport aux électrodes engendre le signal pour la servocommande (37).
